Europäisches Patentamt
European Patent Office
Office européen des brevets

(19)

(11) Publication number: 0 342 849 A2

(12) **EUROPEAN PATENT APPLICATION**

(21) Application number: 89304697.9

(22) Date of filing: 09.05.89

(51) Int. Cl.4: C07C 63/70 , C07C 69/76 , C07C 49/807 , C07C 69/738 , C07C 69/734 , C07C 69/732 , C07C 101/14

Claims for the following Contracting States: ES + GR.

(30) Priority: 19.05.88 US 195986

(43) Date of publication of application: 23.11.89 Bulletin 89/47

(84) Designated Contracting States: AT BE CH DE ES FR GB GR IT LI LU NL SE

(71) Applicant: PFIZER INC.
235 East 42nd Street
New York, N.Y. 10017(US)

(72) Inventor: Busch, Frank R.
6, Pheasant Run Drive
Gales Ferry State of Connecticut(US)
Inventor: Hecker, Scott J.
151 Lamphere Road
Mystic State of Connecticut(US)
Inventor: McGuirk, Paul R.
8, Lynn Drive
Ledyard State of Connecticut(US)
Inventor: O'Neill, Brian T.
1516 Essex Road
Westbrook State of Connecticut(US)
Inventor: Watson, Harry A., Jr.
244 Shennecossett Parkway
Groton State of Connecticut(US)

(74) Representative: Moore, James William, Dr.
Pfizer Limited Ramsgate Road
Sandwich Kent CT13 9NJ(GB)

(54) **Intermediates for preparing 1,4-dihydro-4-oxo-quinoline-3-carboxylic acid esters.**

Compounds of the formula:

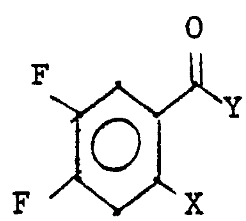

IV

wherein Y is $OR^1$, fluoro, chloro, methyl,

EP 0 342 849 A2

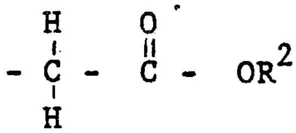

,

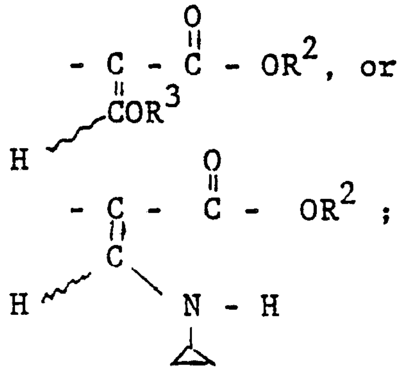

, or

;

$R^1$, and $R^3$ are independently selected from hydrogen and $C_1$-$C_4$ alkyl, $R^2$ is $C_1$-$C_4$ alkyl and X is fluoro, chloro or bromo, with the proviso that when Y is OH, X cannot be bromo and with the proviso that X can be fluoro only if $R^2$ is methyl.

# INTERMEDIATES FOR PREPARING 1 ,4-DIHYDRO-4-OXO-QUINOLINE-3-CARBOXYLIC ACID ESTERS

(57) The present invention relates to esters of 1-cyclopropyl-6,7-difluoro-1,4-dihydro-4-oxo-quinoline-3-carboxylic acid and to processes and intermediates for their preparation. The foregoing compounds are useful in the preparation of 1-cyclopropyl-6-fluoro-1,4-dihydro-4-oxo-7-substituted-quinoline-3-carboxylic acids, which are useful in the treatment of bacterial infections in mammals. Such compounds are disclosed in United States Patent 4,571,396 and European Patent Application Publication Number 0215650.

The present invention relates to compounds of the formula:

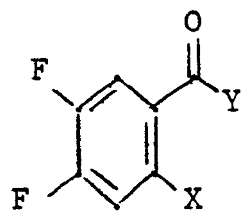

IV

wherein Y is $OR^1$, fluoro, chloro, methyl,

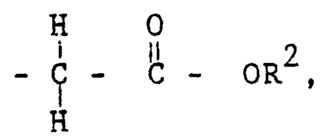

or

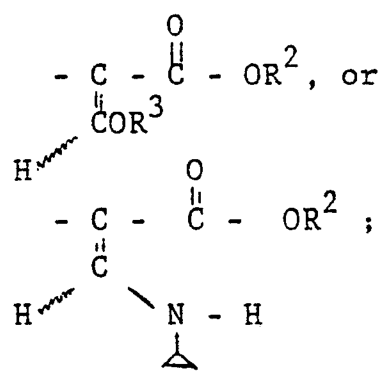

$R^1$ and $R^3$ are independently selected from hydrogen and $C_1$-$C_4$ alkyl, $R^2$ is $C_1$-$C_4$ alkyl and X is fluoro, chloro or bromo, with the proviso that when Y is OH, X cannot be bromo, and with the proviso that X can be fluoro only if $R^2$ is methyl, except that when Y is methyl X cannot be fluoro. It should be noted that certain compounds of the invention (e.g., compound VII shown in scheme I) can also exist in their enol forms and it should be understood that such forms are included by the foregoing representations.

Preferably, X is chloro. It is also preferred that $R^1$ is hydrogen and that $R^2$ and $R^3$ are independently selected from methyl and ethyl. More preferably, X is chloro, $R^1$ is hydrogen and $R^2$ and $R^3$ are independently selected from methyl and ethyl. Most preferably, X is chloro, $R^2$ is methyl, and $R^3$ is methyl or ethyl.

In another preferred embodiment of the present invention, Y is chloro, methyl or hydroxyl and X is chloro.

In another preferred embodiment of the present invention, Y is

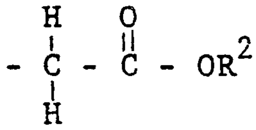, 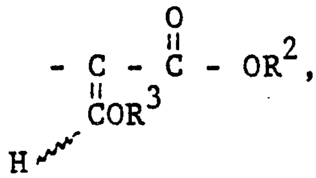, or

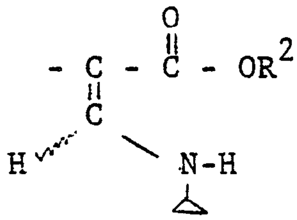

wherein $R^2$ is methyl and $R^3$ is as defined above. In the foregoing embodiment, Y is more preferably

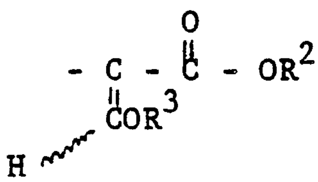 or 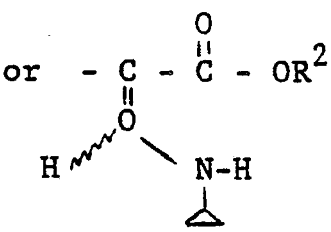

wherein $R^2$ is methyl and $R^3$ is as defined above.

Specific preferred compounds are the following:
2-chloro-4,5-difluorobenzoic acid;
2-chloro-4,5-difluorobenzoyl chloride;
ethyl 3-(2-chloro-4,5-difluoro phenyl-3-oxopropionate;
ethyl 2-(2-chloro-4,5-difluorobenzoyl)-3-ethoxyacrylate;
ethyl 2-(2-chloro-4,5-difluorobenzoyl)-3-(N-cyclopropylamino)acrylate;
2-chloro-4,5-difluoroacetophenone;
methyl 3-(2-chloro-4,5-difluorophenyl)-3-oxopropionate;
methyl 2-(2-chloro-4,5-difluorobenzoyl)-3-ethoxyacrylate; and
methyl 2-(2-chloro-4,5-difluorobenzoyl)-3-(N-cyclopropylamino)-acrylate.

Another embodiment of the present invention relates to a process for preparing monomethyl malonic acid or monoethyl malonic acid comprising reacting a salt of monomethyl or monoethyl malonic acid (e.g., an alkaline metal salt, an alkaline earth metal salt or an ammonium salt) with a strong acid (e.g., hydrochloric acid or sulfuric acid) in water and continuously extracting the product from the aqueous layer with an organic solvent (e.g., methylene chloride).

Another embodiment of the present invention relates to a process for generating the dianion of a mono-($C_1$-$C_4$)alkyl malonic acid (e.g., monomethyl malonic acid or monoethyl malonic acid) comprising reacting said mono($C_1$-$C_4$)alkyl malonic acid with n-butyl lithium in an inert solvent (e.g., tetrahydrofuran) at a temperature of about -20 to about 20° C.

Another embodiment of the present invention relates to a process for preparing a compound of the formula

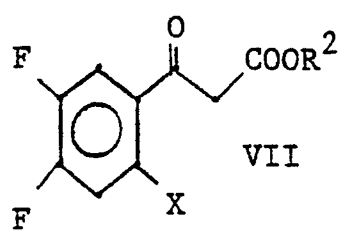

VII

wherein $R^2$ is $C_1$-$C_4$ alkyl and X is fluoro, chloro or bromo, with the proviso that X can be fluoro only when

$R^2$ is methyl, comprising reacting a compound of the formula

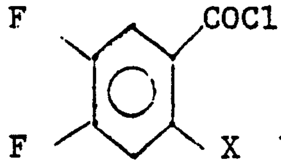

VI

wherein X is as defined with respect to formula VII, with the dilithium salt of mono($C_1$-$C_4$)alkyl malonate (e.g., monomethyl malonate or monoethyl malonate)

Another embodiment of the present invention comprises a process for preparing a compound of the formula I comprising reacting 5,6-difluoro isatin with alkaline hydrogen peroxide.

Another embodiment of the present invention relates to a process for preparing the aforementioned compound of the formula VII comprising reacting a compound of the formula

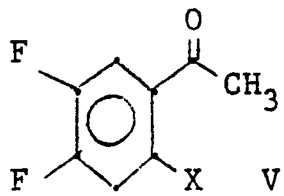

V

wherein X is fluoro, chloro or bromo with a strong base (e.g., a metal hydride such as sodium or potassium hydride or a diisopropylamide anion, such as lithium or potassium diisopropyl amide) and then reacting the resulting anion with a $C_1$-$C_4$ alkyl cyanoformate (e.g., methyl cyanoformate). As used herein, the term strong base refers to a base having a $pK_a$ greater than a $pK_a$ of the protons on the acetyl group.

Another embodiment of the present invention relates to a process for preparing a compound of the formula

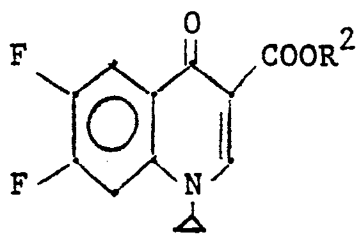

wherein $R^2$ is $C_1$-$C_4$ alkyl comprising reacting a compound of the formula

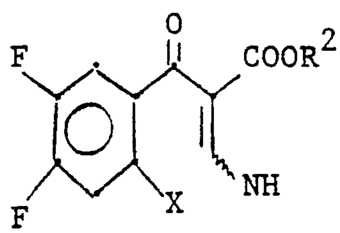

IX

wherein $R^2$ is $C_1$-$C_4$ alkyl, preferably methyl, and X is fluoro, chloro or bromo, with the proviso that X can be fluoro only when $R^2$ is methyl, with a base.

Another embodiment of the present invention relates to a process for preparing said compound of the formula IX comprising reacting a compound of the formula

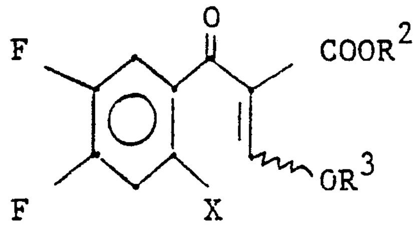

VIII

wherein $R^2$ is methyl or ethyl, preferably methyl, $R^3$ is methyl or ethyl and X is fluoro, chloro or bromo, with the proviso that X can be fluoro only when $R^2$ is methyl, with cyclopropylamine.

In a preferred embodiment of the present invention, the foregoing compound of the formula VIII is reacted with cyclopropylamine in a $C_5$-$C_7$ cycloalkane solvent (preferably, cyclohexane) to produce said compound of the formula IX as a crystalline precipitate. In a more preferred embodiment, $R^2$ is methyl or ethyl; in a still more preferred embodiment, $R^2$ is methyl or ethyl and $R^3$ is methyl or ethyl; and most preferably, $R^2$ is methyl or ethyl, $R^3$ is methyl or ethyl, and X is chloro.

Another embodiment of the present invention comprises a process for preparing the aforementioned compound of the formula VIII, comprising reacting the aforementioned compound of the formula VII with a tri-($C_1$-$C_4$)alkylorthoformate, preferably trimethylorthoformate or triethylorthoformate, in the presence of a $C_1$-$C_4$ alkanoic anhydride, preferably acetic anhydride. In the foregoing process, the group $R^2$ in the compound of the formula VII is preferably methyl.

Another embodiment of the present invention relates to the compound of the formula IV wherein Y is methyl and X is fluoro.

## Detailed Description of the Invention

The following reaction schemes illustrate the preparation of compounds of the present invention and their utility in preparing compounds which are useful in the treatment of bacterial infections in mammals.

Scheme I

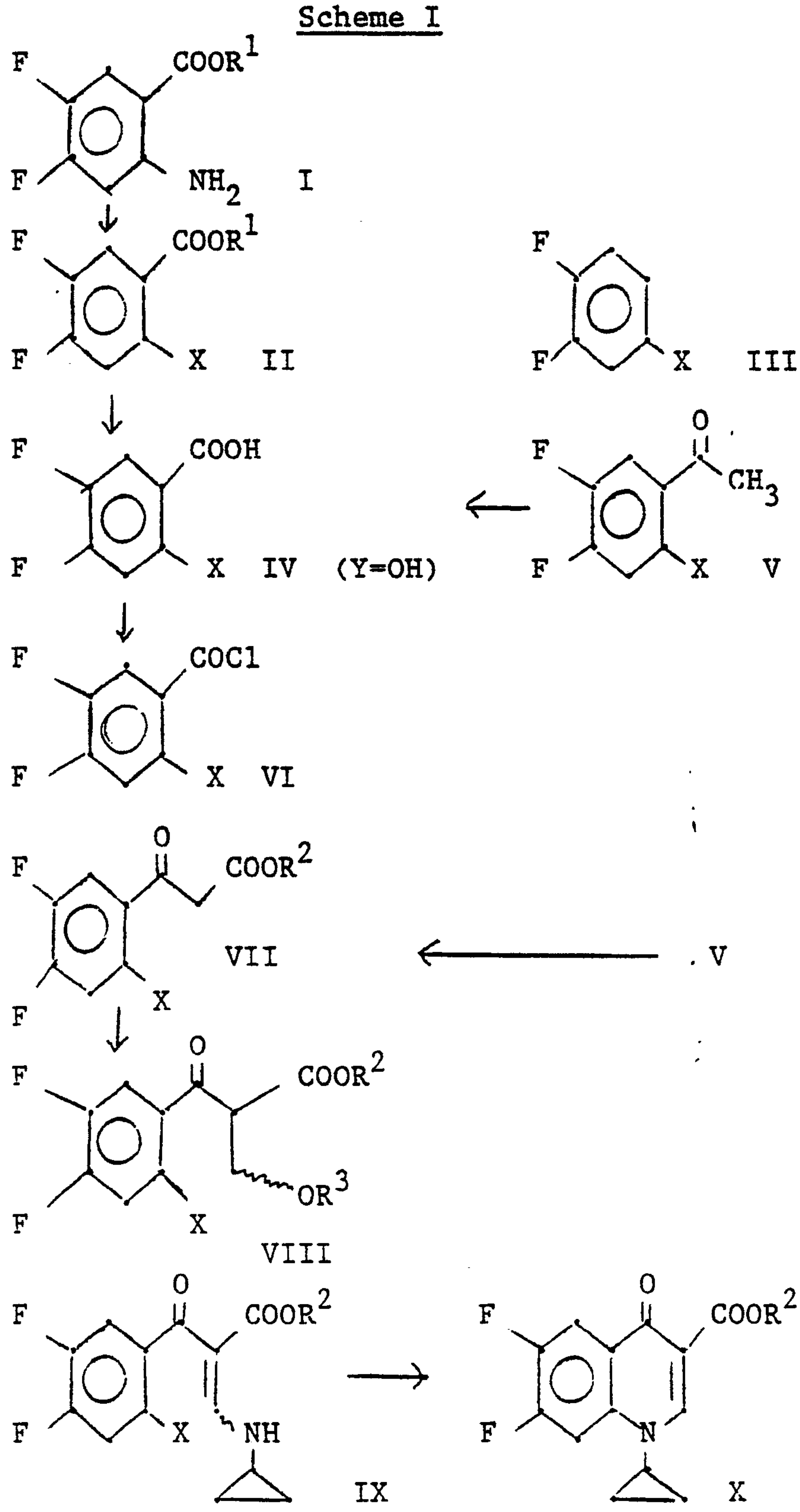

I

II

III

IV (Y=OH)

V

VI

VII

V

VIII

IX

X

## Scheme II

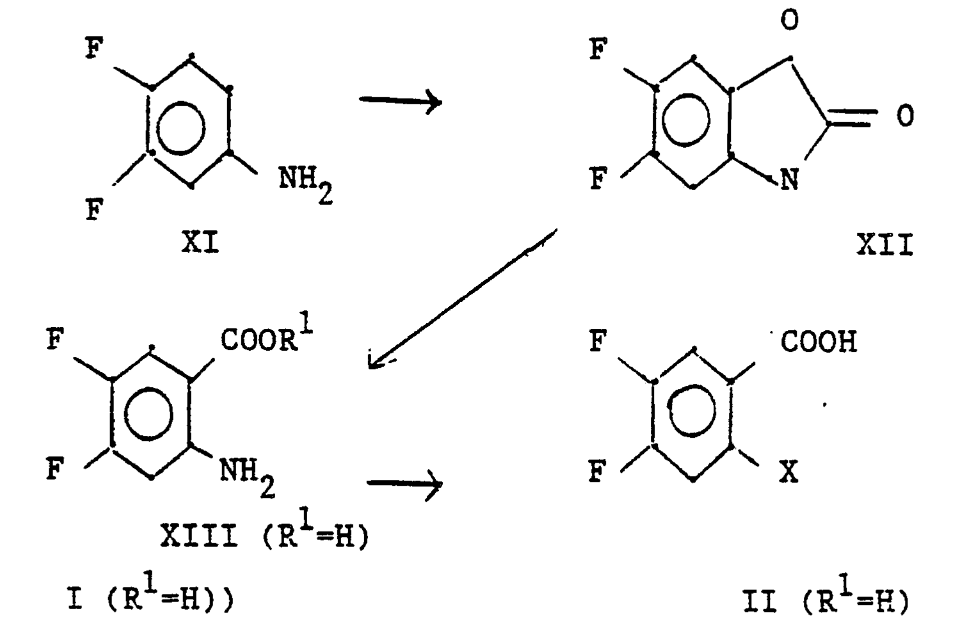

XI

XII

XIII ($R^1$=H)

I ($R^1$=H))

II ($R^1$=H)

## Scheme III

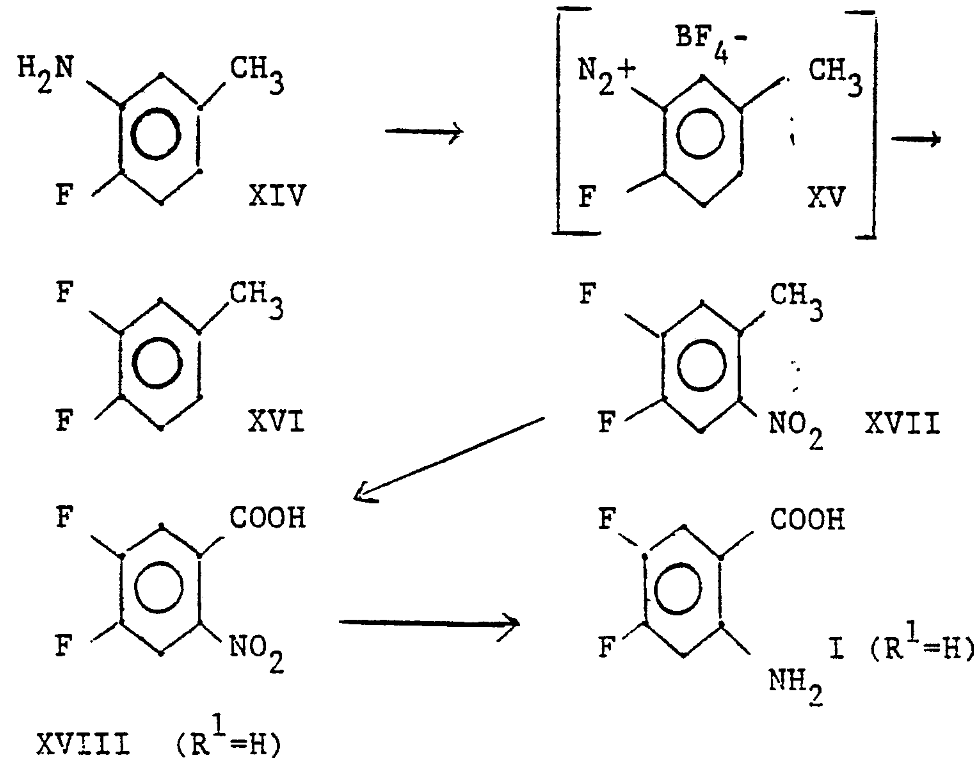

XIV

XV

XVI

XVII

XVIII ($R^1$=H)

I ($R^1$=H)

Scheme I illustrates the preparation of compounds of the formula X from compounds of the formula I or from compounds of the formula III. Typically, the acid IV is converted into the acid chloride VI with a reagent capable of converting an acid to an acid chloride (e.g., thionyl chloride) in toluene, or some other inert solvent such as benzene, hexanes, or methylene chloride, at a temperature between about 50 and about 100° C, preferably at about 80° C, for a period of about 0.5 hours to about 8 hours, preferably about 2 hours. Other reagents useful for acid chloride formation such as oxalyl chloride, phosphorous oxychloride or phosphorous pentachloride may also be utilized. The acid chloride VI may be converted to the substituted benzoyl acetate VII according to the procedure of Wierenga (J. Org. Chem., 41, 310 (1979)) using a $C_1$-$C_4$ ester of malonic acid (e.g., monomethyl malonate or monoethyl malonate) at a temperature of about -70 to

about 20° C, preferably at about -20 to about 20° C. It should be noted that the compound of formula VII can also exist in the enol form which may be represented as

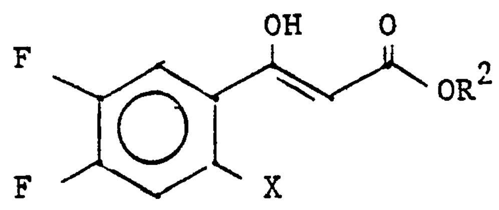

The acid chloride VI may also be reacted with the silyl enol ether derived from a $C_1$-$C_4$ alkyl acetate (e.g., ethyl or methyl acetate), lithium disopropylamide and a silyl chloride (e.g., a trialkyl silyl chloride wherein each alkyl group is independently selected from $C_1$-$C_4$ alkyl and wherein one or more alkyl groups can be replaced by a phenyl group, the preferred silyl chloride being trimethyl chloride) which may be represented as

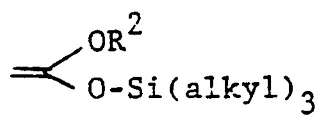

and a Lewis acid catalyst such as zinc chloride, aluminum chloride or titanium chloride. Alternately, as shown in Scheme I, VII may be prepared by reaction of the enolate anion of V, which may be prepared by treatment of V with a strong base such as lithium diisopropylamide, sodium hydride, lithium or potassium hexamethyl disilylazane or potassium t-butoxide, with ethyl or methyl cyanoformate or other carboalkoxylating reagents such as methyl or ethyl chloroformate. Also, VII may be prepared from V according to the procedure of Shahak and Sasson. (Tetrahedron Lett., 4207 (1973)) by reacting the anion of V with carbon disulfide followed by an alkyl iodide (preferably methyl iodide) and hydrolysis. Alternatively, VII may be prepared by conversion of V to its silyl enol ether by quenching the anion of V with trimethylsilyl chloride followed by reaction with a $C_1$-$C_4$ alkyl chloroformate (e.g. methyl or ethyl chloroformate) or other carboalkoxylating reagents (e.g., ethyl or methyl cyanoformate) and an appropriate Lewis acid catalyst such as zinc chloride or titanium tetrachloride in an inert solvent such as toluene, benzene or methylene chloride at a temperature between about -50° C and about +80° C, preferabLy about 25° C. The compound VII is transformed into 2-(2-chloro-4,5-difluoro benzoyl)-3-ethoxy acrylic acid ester (e.g. methyl ester or ethyl ester) (VIII) by the procedure of Grohe et al. (European Patent Application Publication Number 0078362) and is converted to IX using a procedure in Grohe et al. (previous citation). Alternatively, IX is prepared from VIII by dissolving the latter in a $C_5$ to $C_7$ cycloalkane, preferably cyclohexane followed by the addition of cyclopropylamine in a $C_5$ to $C_7$ cycloalkane, preferably cyclohexane. The reaction temperature is controlled between about 20 and about 35° C and stirring is continued for between about 1 and about 2 hours. The product IX is isolated by filtration of the reaction mixture. IX is dissolved in an inert solvent such as tetrahydrofuran, dimethyoxyethane or dimethylformamide and treated with a strong base, as defined above (e.g., potassium tert-butoxide or sodium hydride), at a temperature of about -10° C to about 15° C. After the addition is complete, the reaction mixture is heated to between about 50 and about 150° C to provide X which is 1-cyclopropyl-6,7-difluoro-1,4-dihydro-4-oxo-quinoline-3-carboxylic acid ester (e.g., methyl ester or ethyl ester). Removal of the ester protecting group from compounds of the formula X is conducted via acid hydrolysis, preferably in acetic acid, in the presence of 1N to 4N HCl. The suspension is heated to reflux for about 1.5 to about 3 hours to yield the acid wherein $R^2$ is hydrogen.

Alternatively, compound IV can be obtained from compound III via compound V. Compound III can be acetylated under Friedel-Crafts conditions to give acetophenone V by analogy to the method of W.S. Johnson (J. Am. Chem. Soc., 68, 1648-1650 (1946). Compound V can be oxidized to compound IV using potassium permanganate as described in the foregoing reference or via other oxidative methods.

Scheme II illustrates an alternative synthesis of compounds of the formula II which are intermediates in Scheme I. The 2-amino-4,5-difluoro benzoic acid XIII is dissolved in aqueous acids such as 2N hydrochloric or sulfuric acid at a temperature of about 80 to about 100° C. The resulting solution is then cooled to between about 10° C and about 0° C whereupon a portion of the salt of XIII precipitates. Treatment of the hydrohalide salt of XIII under the standard conditions for diazotiation of amino groups such as addition of an aqueous solution of sodium nitrite yields a diazonium salt. The diazonium salt is not isolated but is added

as a solution over a few minutes to a few hours, depending on the scale, to cuprous chloride in concentrated hydrochloric acid or cuprous bromide in concentrated hydrobromic acid. The product 2-chloro or 2-bromo-4,5-difluoro benzoic acid precipitates from the reaction mixture. The reaction mixture is warmed to ambient temperature between about 20°C and about 30°C and stirred for about 12 to about 18 hours before filtration of the product.

Scheme III illustrates the preparation of the compound of formula I. The scheme is more fully illustrated in the Examples.

Unless indicated otherwise, each of the reactions referred to above may be performed at a pressure of about 0.5 to about 2.0 atmospheres, preferably at ambient pressure (i.e. at about one atmosphere).

In a preferred embodiment of the present invention, the compound of the formula X is prepared from intermediates that are methyl esters rather than ethyl or $C_3$ or $C_4$ esters (see Examples 23, 25, 26 and 27). As shown in Table 1, the compound VIII and IX methyl esters are higher melting and more crystalline than the ethyl esters. Surprisingly, compound IX methyl ester has a melting point that is about 45°C greater than the melting point of the ethyl ester. The higher melting points of the methyl esters makes it easier to isolate and purify those compounds due to their greater crystallinity.

Table 1

| Comparison of Melting Points Of Methyl ($R^2 = CH_3$) Esters and Ethyl ($R^2 = C_2H_5$) Esters | | |
|---|---|---|
| Compound | Ethyl ester m.p. °C | Methyl ester m.p. °C |
| VIII, X = Cl $R^3 = C_2H_5$ | oil | 103-107 |
| IX, X = Cl | 66-68 | 112-113 |

The compound of the formula X may be used to prepare the antibacterial compound 1-cyclopropyl-6-fluoro-1,4-dihydroxy-4-oxo-7-(5-methyl-(1S,4S)-2,5-diazabicyclo[2.2.1]hept-2-yl)-quinoline-3-carboxylic acid, which is disclosed in European Patent Application Publication No. 0215650, by coupling the compound of the formula X wherein R is $C_1$-$C_4$ alkyl with the required side chain followed by hydrolysis or by first hydrolyzing a compound of the formula X wherein R is $C_1$-$C_4$ alkyl to the compound wherein R is hydrogen and then coupling with the required side chain.

The aforementioned antibacterial compound and similar antibacterial compounds that may be prepared using the compound of formula X are useful in the treatment of a broad spectrum of bacterial infections, particularly the treatment of infections caused by gram-positive bacterial strains. The antibacterial compounds may be administered alone, but will generally be administered in admixture with a pharmaceutical carrier selected with regard to the intended route of administration and standard pharmaceutical practice. For example, they can be administered orally or in the form of tablets containing such excipients as starch or lactose, or in capsules either alone or in admixture with excipients, or in the form of elixirs or suspensions containing flavoring or coloring agents. In the case of animals, they are advantageously contained in an animal feed or drinking water in a concentration of 5-5000 ppm, preferably 25-500 ppm. They can be injected parenterally, for example, intramuscularly, intravenously or subcutaneously. For parenteral administration, they are best used in the form of a sterile aqueous solution which can contain other solutes, for example, enough salt or glucose to make the solution isotonic. In the case of animals, the antibacterial compounds can be administered intramuscularly or, subcutaneously at dosage levels of about 0.1-50 mg/kg/day, advantageously 0.2-10 mg/kg/day given in a single daily dose or up to 3 divided doses. The antibacterial compounds can be administered to humans for the treatment of bacterial diseases by either the oral or parenteral routes, and may be administered orally at dosage levels of about 0.1 to 500 mg/kg/day, advantageously 0.5-50 mg/kg/day given in a single dose or up to 3 divided doses. For intramuscular or intravenous administration, dosage levels are about 0.1-200 mg/kg/day, advantageously 0.5-50 mg/kg/day. While intramuscularly administration may be a single dose or up to 3 divided doses, intravenous administration can include a continuous drip. Variations will necessarily occur depending on the weight and condition of the subject being treated and the particular route of administration chosen as will be known to those skilled in the art. The antibacterial activity of the compounds is shown by testing according to the Steer's replicator technique which is a standard <u>in vitro</u> bacterial testing method described by E.

Steers et al., Antibiotics and Chemotherapy, 9, 307 (1959).

The following Examples illustrate the preparation of the compounds of the present invention. All melting points referred to in the Examples are uncorrected.

## Example 1

### 3,4-Difluoroisonitrosoacetanilide

To a 5 liter four neck round bottom flask equipped with a mechanical stirrer and a condenser was charged a solution of 145 g of chloral hydrate in 1.920 liters of water. Then 930 g of sodium sulfate was slowly added with rapid stirring. To this mixture was then added a solution of 128 g 3,4-difluoroaniline in 67.6 ml of 12 M hydrochloric acid and 527 ml of water. Then, a solution of hydroxylamine hydrochloride (173 g) in 800 ml $H_2O$ was added. The mixture (an off tan/orange slurry) was heated rapidly to 95° C on a steam bath. The slurry thinned out at 80° C but never became a homogenous solution. The mixture was stirred rapidly at 95° C for 20 minutes and then removed from the steam bath and allowed to cool gradually overnight to room temperature. The solids were filtered and washed with water and then dried at 45° C for about two days to obtain 178.5 g (greater than 100% presumably due to the presence of residual solvent) of the title compound, m.p. 149-151° C.

## Example 2

### 5,6-Difluoroisatin

To a 2 liter four neck flask equipped with a mechanical stirrer, condenser, thermometer and and nitrogen atmosphere was charged 890 ml of concentrated sulfuric acid. This was heated to 85° C on a steam bath. To this was charged in small aliquots 178 g of 3,4-difluoroisonitrosoacetanilide while the reaction temperature was maintained at 85-90° C. The reaction mixture was stirred for 30 minutes at 85-90° C and then allowed to cool to 25° C. The reaction mixture was then quenched into a 2.5 liters of a mixture of water and ice. A precipitate was formed after addition was complete. The mixture was stirred for 1 hour and the suspension was then filtered and washed with water. The solids were dried in a vacuum oven at 45° C and a pressure of 20 mg Hg overnight to obtain 138 g of the title compound, m.p. 221-222° C.

## Example 3

### 2-Amino-4,5-difluorobenzoic acid

To a 2 liter four neck round bottom flask equipped with a mechanical stirrer, a condenser and a thermometer was charged 100 g of 5,6-difluoroisatin and 900 ml of aqueous 2.5N sodium hydroxide solution. The mixture was stirred until a homogeneous purple solution resulted. To this solution, and with external cooling (acetone/wet ice), was charged 162 ml of a 30% solution of hydrogen peroxide over a period of about 20 minutes. The reaction temperature never rose above 45° C throughout the addition.

After addition was complete, the reaction mixture was heated for 2 hours and was then cooled to room temperature. This mixture was then carefully quenched into a solution of 6N HCl (200 ml) in a 5 liter flask. The temperature stayed at about 15-20° C throughout the addition. A 5 liter flask was used because of extensive foaming during the quench. The pH of the quench mixture was adjusted from about 5.0 to about

1.0 with concentrated hydrochloric acid as precipitation of the product caused stirring to slow down.

To the reaction mixture was then added 260 ml of 50% NaOH solution to bring the pH up to 4.50. The mixture was filtered and the solids were sucked dry. The product was then dried in an oven at 40 °C and reduced pressure to obtain 95 g of the title compound, m.p. 180-181 °C. NMR($CDCl_3$ 300 MHz) : 7.56 (1H,dd,J=8 Hz, J=7 Hz), 6.68 (1H,dd,J=8 Hz, J=7 Hz).

## Example 4

### 2-Chloro-4,5-difluorobenzoic acid

To a 25 ml flask equipped with magnetic stir bar and nitrogen atmosphere was charged 500 mg of 2-amino-4,5-difluorobenzoic acid and 4.6 ml of 2.0N aqueous hydrochloric acid. The mixture was heated on a hot water bath to dissolve the acid (HCl-salt form). An amber solution resulted at elevated temperature. The solution was allowed to cool at room temperature. Some material precipitated from the solution. The solution was cooled to -3 °C with an acetone/wet ice bath. To the cooled mixture was charged, under the reaction mixture surface, a solution consisting of 200 mg of sodium nitrate/1.0 ml $H_2O$. During such addition, the reaction temperature was not allowed to rise about 0 °C. Addition required about 3 minutes. The mixture was stirred for 5 minutes and then tested for excess nitrate with potassium-iodide-starch paper. An immediate blue color resulted (tests positive).

The mixture was then charged to a solution of 430 mg CuCl in 1.5 ml of concentrated HCl at -3 °C. Addition was performed in small aliqucts so that foaming was controlled. Addition required 15 minutes and the reaction temperature never rose above 0 °C throughout addition. After the addition was complete, the reaction mixture was stirred at 0 °C for about 5 minutes and then allowed to warm to room temperature with rapid stirring.

The reaction was allowed to proceed overnight and the mixture was then filtered affording 0.26 g of the title compound, m.p. 101-102 °C. NMR ($CDCl_3$, 300 MHz) : 7.90 (1H,dd,J=7 Hz, J=8 Hz), 7.32 (1H,dd,J=7 Hz, J=8 Hz).

## Example 5

### 2-Chloro-4,5-difluorobenzoyl chloride

A 5 liter flask was charged with 930 ml of toluene, 200 g (1.04 mole) of 2-chloro-4,5-difluorobenzoic acid, and 2.2 g (0.03 mole, 0.03 eq) of pyridine. This mixture was heated to 60 °C and a solution resulted. While maintaining a pot temperature of 60-70 °C, 161.4 g (1.4 mole, 1.3 eq) of thionyl chloride was added over 40 minutes. The reaction was then heated to reflux for 30 minutes and was then concentrated to an oil. The oil was chased twice under vacuum to an oil with fresh toluene. This gave 201.8 g (92.1% yield) of the title compound.

## Example 6

### Ethyl 3-(2-chloro-4,5-difluorophenyl)-3-oxopropionate

A 12 liter flask was charged with 252 g of monoethylmalonic acid (2.2 eq) and 4 liter of THF (tetrahydrofuran). The resulting solution was cooled to below -10 °C and stirred under nitrogen and 2.3 liters of 1.6 molar n-butyl lithium (3.81 mole, 4.4 eq) in hexane was added over 4 hours at -5 to -10 °C. At the

same temperature, a solution of 183.1 g of the title compound of Example 5 (0.867 mole, 1.0 eq) in 900 ml of THF was added over one hour. The reaction was quenched onto 7.15 liters of 2N HCl and extracted with $CH_2Cl_2$ (1x2 liters, 1x4 liters). The organic layer were combined and washed first with saturated $NaHCO_3$ - (2x3 liters) then with saturated NaCl (1x2.5 liters). After drying over $MgSO_4$, and then filtering, the solvent was stripped to an oil. The product was crystallized from 300 ml of isopropyl alcohol at 5° C, filtered, and then vacuum dried at 20° C to give 152.8 g (67.1% yield) of the title compound, m.p. 52-53° C.

## Example 7

### Ethyl 2-(2-chloro-4,5-difluorobenzoyl)-3-ethoxy-acrylate

A one liter flask was charged with 100 g of the title compound of Example 6 (0.38 mole), 84.4 g of triethylorthoformate (0.57 mole, 1.5 eq),and 97 g of acetic anhydride (0.95 mole, 2.5 eq). This mixture was heated to reflux for 1.5 hours, at which time TLC (thin layer chromatography) on 3:1 hexane: ethyl acetate showed that the reaction was complete. The amber solution was vacuum stripped to yield the title compound as an oil.

## Example 8

### Ethyl 2-(2-chloro-4,5-difluorobenzoyl)-3-(N-cyclopropylamino)-acrylate

A one liter flask was charged with the title compound of Example 8 (0.381 mole) crude and cyclohexane was added (3x200 ml), each time vacuum stripping to an oil. This was done to azeotrope any acetic anhydride present. The resulting oil was diluted to 400 ml with cyclohexane. Over 40 minutes, a solution of 24 g (0.42 mole, 1.1 eq) cyclopropylamine in 29 ml of cyclohexane was added at less than 35° C. The reaction mixture was stirred for 1.5 hours during that time and the product precipitated. The flask was immersed in an ice water bath and the product allowed to granulate for 30 minutes. The product was filtered and air dried to give 98.5 g (78.5% yield) of the title compound, m.p. 70-73° C.

## EXAMPLE 9

### 1-Cyclopropyl-6,7-difluoro-1,4-dihydro-4-oxo-quinoline-3-carboxylic acid ethyl ester

A 3 liter flask was charged with 50 g of the title compound of Example 8 (0.15 mole) and 1 liter of THF. This solution was stirred at less than 10° C while 18.7 g (0 .14 mole, 1.1 eq) of potassium tert-butoxide was added over 15 minutes. The reaction was then heated to reflux for 1.5 hours, at which time TLC in 4:1 ethyl acetate: hexane showed that the reaction was complete. The reaction was then quenched by adding one liter of ice water. The product was collected by filtration and air dried at 45° C to give 34.2 g (77.2% yield) of the title compound, m.p. 226-227.5° C.

## EXAMPLE 10

### 1-Cyclopropyl-6,7-difluoro-1,4-dihydro-4-oxo-quinoline-3-carboxylic acid

A 2 liter flask was charged with 50 g (0.17 mole) of the title compound of Example 9, 322 ml of 1N HCl, and 263 ml of acetic acid. This suspension was heated to reflux for 1.5 hours, at which time TLC in 9:1 chloroform:methanol showed that the reaction was complete. The suspension was then cooled to below 50° C, and diluted with 570 ml of ice water slurry. The suspension was granulated for 30 minutes at below 10° C, filtered, washed with water, and then air dried at 45° C, to give 43.4 g (96.8% yield) of the title compound.

## EXAMPLE 11

### 1-Cyclopropyl-6-fluoro-1,4-dihydro-4-oxo-7-(5-methyl-(1S,4S)-2,5-diazabicyclo[2.2.1]hept-2-yl)-quinoline-3-carboxylic acid

A one liter flask was charged with 30 g of the title compound of Example 10 (0.113 mole), 25.1 g of (1S, 4S), 5-methyl-2,5-diazabicyclo[2.2.1]heptane dihydrochloride (0.136 mole 1.2 eq), and 180 ml of pyridine. This suspension was stirred while 44.7 g (.0294 mole, 2.6 eq) of 1,8-diazabicyclo[5.4.0]undec-7-ene was added. The reaction was heated at 85° C for 3 hours, cooled to room temperature, and the product was filtered and washed with absolute ethanol (2x50 ml). The separated solids were vacuum dried at 50° C to give 34.0 g (84.3% yield) of the title compound, m.p. 268-272° C.

## EXAMPLE 12

### 2-Chloro-4,5-difluoroacetophonone

To a stirring mixture of 1-chloro-3,4-difluorobenzene (3.83 g., 25.8 mmol) and acetyl chloride (4.31 g, 54.9 mmol) under a nitrogen atmosphere was added, in several portions, aluminum chloride (7.51 g, 56.3 mmol), allowing the bubbling to subside between additions. The resulting mixture was heated at 85° C for 18 hours and was poured into a stirring mixture of water (100 ml) and dichloromethane (100 ml). The organic layer was washed with water (50 ml) saturated sodium bicarbonate solution (50 ml), and brine (50 ml), and was dried ($MgSo_4$) and filtered. The solvent was removed with a rotary evaporator, and the crude product was distilled, affording 2.50 g (53%) of the title compound as a clear, colorless oil b.p. 60-70° C/2mm.

## EXAMPLE 13

### 2-Chloro-4,5-difluorobenzoic acid

To a stirring solution of 2-chloro-4,5-difluoroacetophenone (2.60 g, 13.6 mmol) in dioxane (30 ml) add dropwise a solution of sodium hypobromite (prepared at 0° C by dissolving 8.5 g of sodium hydroxide and 3.1 g of bromine in 40 ml of water). Upon completion of the addition, stir the mixture for 15 minutes and add sodium bisulfite to destroy the excess hypobromite. Acidify the mixture with 1N HCl, and then extract with dichloromethane (2 x 50 ml), dry ($MgSO_4$), and filter. Remove the solvent with a rotary evaporator to afford the title compound.

EXAMPLE 14

2-Bromo-4,5-difluorobenzoic acid

A 200 ml 3-necked flask was charged with 10.5 ml (0.10 mole) of 1-bromo-3,4-difluorobenzene and 8.5 ml (0.15 mole, 1.5 eq) of acetyl chloride. The mixture was cooled and 33.4 g (0.25 moles) of aluminum chloride was added. The mixture was stirred and heated to 120° C for 2 hours. HCl was evolved and the mixture turned dark in color. TLC on silicia gel (9:1 hexane:ethyl acetate) showed that the starting material was consumed. The reaction mixture was cooled and quenched with 250 g of ice. The aqueous layer was then extracted three times with 100 ml portions of methylene chloride. The 2-bromo-4,5-difluoroacetophenone intermediate was obtained by removing the methylene chloride at reduced pressure. The intermediate was used directly by treatment with 225 ml of industrial bleach solution and heating the mixture to reflux for 2 hours. The organic layer was decanted and the aqueous layer was quenched with sodium bisulfite. The pH of the aqueous layer was then adjusted to 1.0 with concentrated hydrochloric acid. The product precipitated and was recovered by filtration. This crude product was purified by dissolving it in 100 ml of water adjusted to pH 10.3, filtering, lowering the pH to 1.4 and then filtering to obtain the title compound (9.5 g, 50% yield), mp 102-104° C.

EXAMPLE 15

Ethyl 3-(2-chloro-4,5-difluorophenyl)-3-oxopropionate

To a stirring solution of diisopropylamine (0.70 ml, 5.0 mmol) in THF (10 ml) at 0° C was added a solution of n-butyllithium in hexane (2.0 ml, 2.5 M, 5.0 mmol). After 10 minutes, the solution was cooled to -78° C and a solution of 2-chloro-4,5-difluoroacetophenone (784 mg, 4.11 mmol) in THF (2 ml) was then added. After 30 minutes at -78°, ethyl cyanoformate (594 mg, 6.0 mmol) was added. The mixture was then stirred for 5 minutes at -78° C and for 15 minutes at 0° C. Ice and 1N HCl (10 ml) were added, and the mixture was diluted with ethyl ether (20 ml). The organic layer was separated, washed with water (10 ml), saturated aqueous sodium bicarbonate solution (10 ml) and brine (10 ml), and was dried ($MgSO_4$) and filtered. The solvent was removed with a rotary evaporator, and the crude product was subjected to column chromatography on silica gel (using 9:1 hexane/ethyl acetate as the eluant) to afford 658 mg (61%) of the title compound as a colorless oil, the $^1H$ NMR spectrum of which showed it to be a mixture of keto and enol tautometers.

EXAMPLE 16

2-Fluoro-5-methyl-benzenediazonium tetrafluoroborate

To 6 ml of 12N HCl was added 2-fluoro-5-methylaniline (3.38 g, 27.5 mmol). A dense solid precipitated, and water (5 ml) was added in order to bring the salt into solution. After cooling to 0° C, a solution of $NaNO_2$ (1.90 g, 27.5 mmol) in water (5 ml) was added slowly, and additional water was added to afford efficient stirring. After 15 minutes, a solution of $NaBF_4$ (4.28 g, 39.0 mmol) in water (10 ml) was added, and a precipitate formed. The mixture was stirred for 30 minutes at 0° C, and was filtered. The precipitate was washed with ice-cold water, methanol and ether, and was air-dried to afford 4.47 g (74%) of the title compound as a fluffy white solid, m.p. 139-143° dec.

EXAMPLE 17

3,4-Difluorotoluene

The diazonium salt prepared in Example 16 was placed in a 250 ml round-bottom flask topped with a reflux condenser in a fume hood. The condenser was equipped with a gas inlet tube connected to a length of tubing running to the back of the fume hood. The surface of the flask was heated with a heat gun, and the solid was gradually converted to a refluxing liquid. When all of the solid was liquified, the glassware was rinsed with dichloromethane, and the resulting crude mixture was fractionally distilled at atmospheric pressure, affording 1.76 g (69%) of the title compound as a colorless liquid, b.p. 124-128°.

EXAMPLE 18

4,5-Difluoro-2-nitrotoluene

To a stirring solution of 3,4-difluorotoluene (1.34 g, 10.5 mmol) in a mixture of concentrated sulfuric acid (1.7 ml) and acetic acid (1 ml) at 0° C was added dropwise a chilled mixture of concentrated nitric acid (1.6 ml) and concentrated sulfuric acid (3.4 ml). After stirring for 10 minutes at 0° C, ice (20 g) and dichloromethane (30 ml) were added. The layers were then separated and the organic phase was washed with water (20 ml) and brine (20 ml), and was dried ($MgSO_4$) and filtered. The solvent was removed from the organic layer with a rotary evaporator, affording 1.73 g (95%) of the title compound as a light yellow oil $^1$H NMR ($CDCl_3$) : 7.91 (1H, dd, J = 8, 10), 7.14 (1H, dd, J = 8, 10), 2.57 (3H, s).

EXAMPLE 19

4,5-Difluoro-2-nitrobenzoic acid

To a solution of 4,5-difluoro-2-nitrotoluene (108 mg, 0.63 mmol) in acetic acid (1 ml) was added $CrO_3$ - (286 mg, 2.86 mmol) followed by water (0.5 ml). When all of the $CrO_3$ had dissolved, concentrated sulfuric acid (0.5 ml) was added dropwise, at which point an exothermic reaction took place. The resulting mixture was heated at 55° for 1 hour, and water (10 ml) and ethyl acetate (20 ml) were then added. The organic layer was separated, washed with water (10 ml) and brine (10 ml), and was dried ($MgSO_4$) and filtered. The solvent was removed from the organic layer with a rotary evaporator, and the crude product was recrystallized from toluene, affording 78 mg (61%) of the title compound as a white solid, m.p. 152-153.5° C.

EXAMPLE 20

2-Amino-4,5-difluorobenzoic acid

To a solution of 4,5-difluoro-2-nitrobenzoic acid (415 mg. 2.04 mmol) in ethanol (20 ml) in a Parr bottle was added 500 mg of Raney nickel (obtained from 1 ml of commercially available 50% suspension in water, freshly washed with water and ethanol). The mixture was placed in a Parr shaker under a hydrogen

atmosphere at 30 psi for 1 hour, and was then filtered. The solvent was removed with a rotary evaporator, affording 334 mg (95%) of the title compound as a white solid, m.p. 178-182°C dec.

EXAMPLE 21

1-Cylopropyl-6-fluoro-1,4-dihydro-4-oxo-7- (S-methyl (1S,4s)-2,5-diazabicyclo[2.2.1]hept-2-yl)-1-quinoline-3-carboxylic acid ethyl ester

1-Cyclopropyl-6,7-difluoro-1,4-dihydro-4-oxo-quinoline-3-carboxylic acid ethyl ester (0.92 g, 3.2 mmoles) and 1.2 equivalents of 5-methyl-3,5-diazabicyclo[2.2.1]heptane dihydrochloride (0.70 g, 3.79 mmoles)was suspended in 5 ml of dry dimethylsulfoxide. To this suspension was added 1,8-diazabicyclo[5.4.0]undec-7-ene (1.2 g, 7.8 mmoles). The mixture was heated to 110°C, at which time a solution resulted. After 18 hours at 110°C, the reaction mixture was cooled to room temperature and poured into water. The resulting mixture was extracted with chloroform. The chloroform layer was then extracted with pH 1 water. The water layer was washed two times with chloroform and then basified with saturated sodium bicarbonate. The desired product was then extracted into chloroform (3 times with 75 ml) and the combined chloroform extracts were dried over sodium sulfate, filtered and concentrated in vacuo to give 1.0 g (85%) of the title compound as a white solid, m.p. 208-211°C.

EXAMPLE 22

1-Cyclopropyl-6-fluoro-1,4-dihydro-4-oxo-7-(5-methyl-(1S,4S)-2,5-diazabicyclo[2.2.1]hept-2-yl)-quinoline-3-carboxylic acid

The title compound from Example 21 (0.97 g, 2.52 mmoles) was suspended in 5 ml of 1N sodium hydroxide and 2 ml of THF. The mixture was heated to a reflux at, which time a solution resulted. The clear yellow solution was heated for 2 hours, cooled to room temperature, neutralized with 1 N HCl and extracted with chloroform (3 x 100 ml). The chloroform extract was dried over sodium sulfate, filtered, and concentrated in vacuo to give 0.97 g of a pale yellow solid (107%, greater than 100% presumably due to residual solvent) which was pure by TLC, m.p. 268-273°C.

EXAMPLE 23

Methyl 3-(2-chloro-4,5-difluorophenyl)-3-oxopropionate

In a 500 ml flask was charged 158 ml of THF and 10 g (0.0847 mol) of monomethyl malonic acid and the solution was cooled to -78°C. To this solution was added dropwise 47.2 ml of 1.6M n-butyl lithium (0.075 mol, 2.2 eq) over 35 minutes. The reaction was then warmed to -20°C and another 47.2 ml of n-butyl lithium (0.075 mol, 2.2 eq) added. The yellow suspension was then cooled to -65°C, and a solution of 7.26 g (0.034 mol) of 2-chloro-4,5-difluorobenzoic acid chloride was added. The reaction mixture was allowed to warm to -20°C and was then quenched into 283 ml of 2N HCl, extracted with 3 x 80 ml of $CH_2Cl_2$, back washed with 3 x 50 ml of saturated sodium bicarbonate solution and 100 ml of brine, and stripped to a solid, 6.7 g, (77%), m.p. 37-44°C. Recrystallization from isopropyl alcohol gave 4.5 g of the title compound, m.p. 51-54°C. The acid chloride used as the starting material in this Example was 80% pure by NMR. The yield of recrystallized material was therefore 66.6%.

## EXAMPLE 24

### Monomethyl malonic acid

A 4 liter flask was changed with 90 g (0.576 moles) of methyl malonate potassium salt, about 540 ml of 1N HCl (to a pH of 1.0), and 2 liters of methylene chloride. The solution was transferred to a continuous extractor which contained an additional two liters of methylene chloride. After 4 hours extraction, no product remained in the aqueous layer (TLC on silica gel with 1:1 hexane:ethyl acetate) and the product layer was separated and dried over $MgSO_4$. Removal of the solvent at reduced pressure gave 57.7 g (84.8% yield) of the title compound. Alternatively, prepare the title compound by substituting the sodium salt for the potassium salt.

## EXAMPLE 25

### Methyl 2-(2-chloro-4,5-difluorobenzoyl)-3-ethoxy-acrylate

In a 25 ml flask was charged 4.0 g (0.016 mol) of the title compound of Example 23, 3.5 g (0.24 mol) of triethyl orthoformate, and 3.78 ml (0.04 mol) of acetic anhydride, and heated to reflux for 2.5 hours. The resulting dark yellow solution was concentrated to 6.12 g of a yellow oil, which crystallized upon standing. This material was repulped in hexane to yield 2.7 g of the title compound, m.p. 103-107° C (56%).

## EXAMPLE 26

### Methyl 2-(2-chloro-4,5-difluorobenzoyl)-3-(N-cyclopropylamino)-acrylate

In a 25 ml flask was added 7 ml of $CH_2Cl_2$ and 2.0 g (0.00656 mol) of the title compound of Example 25 and cooled to -5° C. A solution of cyclopropylamine 0.412 g (0.0072 mol) in 2 ml of $CH_2Cl_2$ was then added dropwise over 11 minutes. After an additional 1 hour of stirring, the reaction mixture was concentrated to a solid, granulated in 5 ml of hexane, and filtered and dried to yield 1.7 g (85%) of the title compound, m.p. 112-113° C.

## EXAMPLE 27

### 1-Cyclopropyl-6,7-difluoro-1,4-dihydro-4-oxo-quinoline-3-carboxylic acid methyl ester

In a 100 ml flask was charged 1.5 g (0.00475 mol) of the title compound of Example 26 and 30 ml of THF. To the cooled solution (2° C), was added portion wise potassium t-butoxide 0.533 g (0.00475 mol) over 2.5 minutes, and stirring continued for another 20 minutes at less than 7° C. The reaction mixture was then heated to reflux for 4 hours, cooled to 45° C, quenched with 30 ml of ice water, granulated for 20 minutes at 5-10° C, filtered, and then washed with cold water. The resulting solid was vacuum dried to yield 1.0 g (77%) of the title compound, m.p. 226-228° C.

## Claims

1. A compound of the formula:

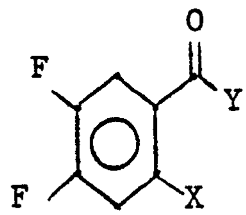

IV

wherein Y is $OR^1$, chloro, methyl,

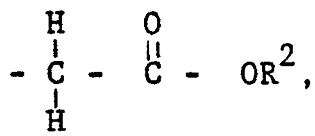

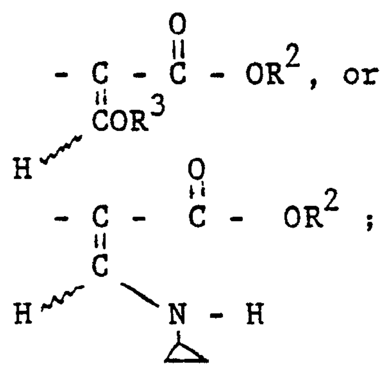

or

$R^1$, and $R^3$ are independently selected from hydrogen and $C_1$-$C_4$ alkyl, $R^2$ is $C_1$-$C_4$ alkyl and X is fluoro, chloro or bromo, with the proviso that when Y is OH, X cannot be bromo, and with the proviso that X can be fluoro only if $R^2$ is methyl, except that X cannot be fluoro if Y is methyl.

2. A compound according to claim 1, characterized in that X is chloro.

3. A compound according to claim 1 or 2, characterized in that $R^1$ is hydrogen and $R^2$ and $R^3$ are independently methyl or ethyl.

4. A compound according to claim 1 or 2, characterized in that $R^1$, $R^2$ and $R^3$ are independently methyl or ethyl.

5. A compound according to any one of claims 1 to 3, characterized in that Y is chloro, methyl or OH.

6. A compound according to claim 1 characterized in that Y is

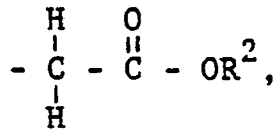

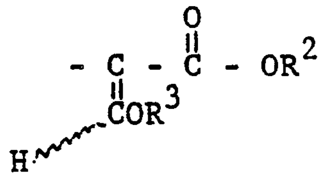

or

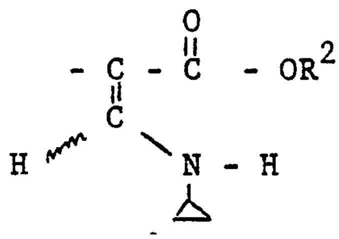

wherein $R^2$ is methyl, and $R^3$ is hydrogen or $C_1$-$C_4$ alkyl.

7. A compound according to claim 1, said compound being selected from the group consisting of
2-chloro-4,5-difluorobenzoic acid;
2-chloro-4,5-difluorobenzoyl chloride;
ethyl 3-(2-chloro-4,5-difluoro phenyl-3-oxopropionate;
ethyl 2-(2-chloro-4,5-difluorobenzoyl)-3-ethoxyacrylate;
ethyl 2-(2-chloro-4,5-difluorobenzoyl)-3-(N-cyclopropylamino)acrylate;
2-chloro-4,5-difluoroacetophenone;
methyl 3-(2-chloro-4,5-difluorophenyl)-3-oxopropionate;
methyl 2-(2-chloro-4,5-difluorobenzoyl)-3-ethoxyacrylate; and
methyl 2-(2-chloro-4,5-difluorobenzoyl)-3-(N-cyclopropylamino)-acrylate.

8. A process for preparing monomethyl malonic acid or monoethyl malonic acid comprising reacting a salt of monomethyl malonic acid or monoethyl malonic acid with a mineral acid in water and continuously extracting the product from the aqueous layer with an organic solvent.

9. A process for generating the dianion of a mono($C_1$-$C_4$)alkyl malonic acid comprising reacting said mono($C_1$-$C_4$)alkyl malonic acid with n-butyl lithium in an inert solvent at a temperature of about -20 to about 20 °C.

10. A process for preparing a compound of the formula:

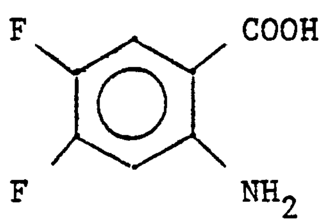

comprising reacting 5,6-difluoro isatin with alkaline hydrogen peroxide.

11. A process for preparing a compound of the formula:

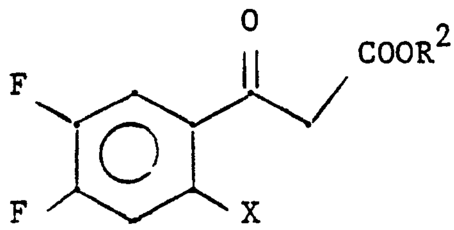

VII

wherein $R^2$ is $C_1$-$C_4$ alkyl and X is fluoro, chloro or bromo, with the proviso that X can be fluoro only when $R^2$ is methyl, comprising reacting a compound of the formula:

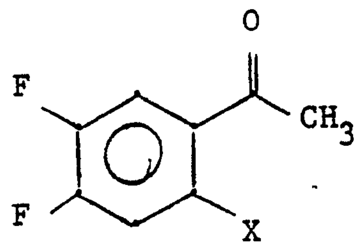

V

wherein X is as defined above with a strong base and then reacting the resulting anion with $C_1$-$C_4$ alkyl cyanoformate.

12. A process for preparing a compound of the formula:

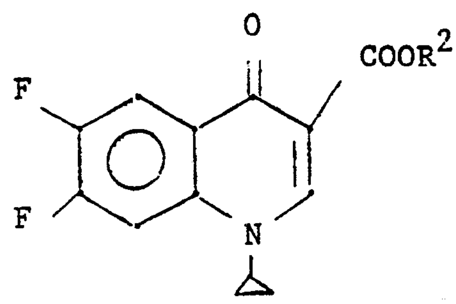

X

wherein $R^2$ is methyl comprising reacting a compound of the formula

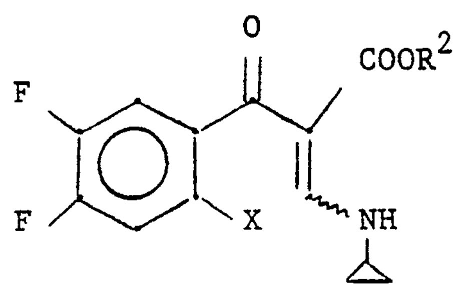

IX

wherein $R^2$ is methyl and X is fluoro, chloro or bromo with a base.

13. A process according to claim 15, wherein said compound of the formula IX is prepared by reacting a compound of the formula

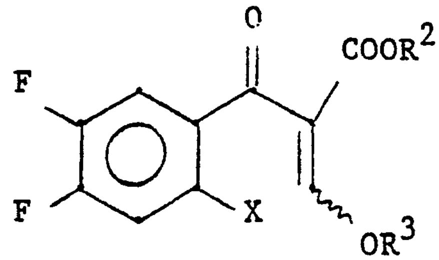

VIII

wherein $R^2$ is methyl, $R^3$ is methyl or ethyl and X is fluoro, chloro or bromo, with cyclopropylamine.

14. A process according to claim 16 wherein said compound of the formula VIII is prepared by reacting a compound of the formula:

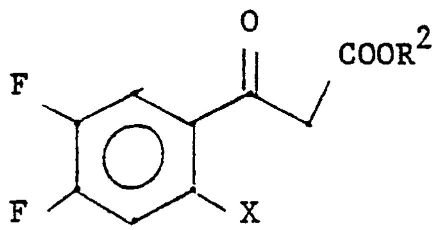

VII

wherein $R^2$ is methyl and X is fluoro, chloro or bromo, with tri($C_1$-$C_4$)alkylorthoformate in the presence of a $C_1$-$C_4$ alkanoic anhydride.

15. A process for preparing a compound of the formula:

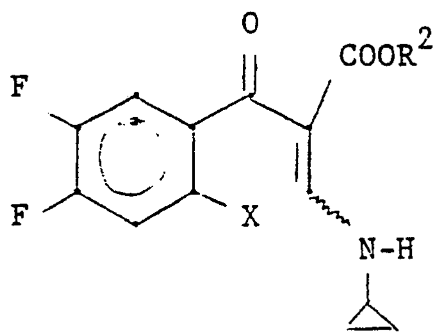

IX

wherein $R^2$ is $C_1$-$C_4$ alkyl, and X is fluoro, chloro or bromo with the proviso that X can be fluoro only when $R^2$ is methyl, comprising reacting a compound of the formula

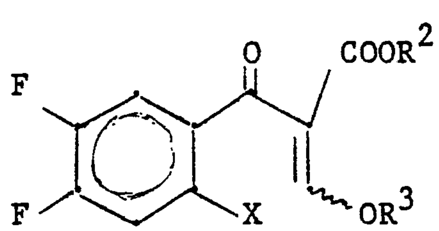

VIII

wherein $R^2$ and X are as defined above and $R^3$ is $C_1$-$C_4$ alkyl, with cyclopropylamine in a $C_5$-$C_7$ cycloalkane to produce said compound of the formula VIII as a crystalline precipitate.

16. A process for preparing a compound of the formula

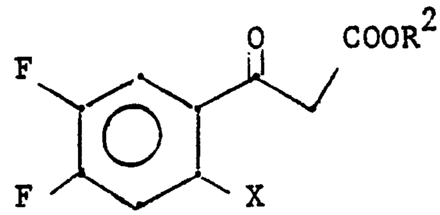

VII

wherein $R^2$ is $C_1$-$C_4$ alkyl and X is fluoro, chloro or bromo, with the proviso that X can be fluoro only when $R^2$ is methyl, comprising reacting a compound of the formula

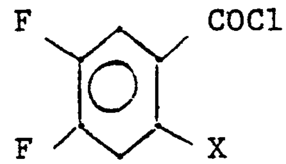

VI

wherein X is as defined above with the dilithium salt of a mono($C_1$-$C_4$)alkyl malonate.

Claims for the following Contracting States: ES, GR

1. A process for preparing a compound of the formula:

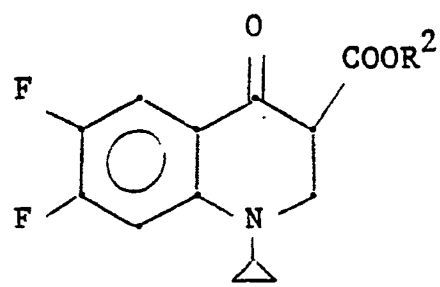

X

wherein $R^2$ is methyl characterized by reacting a compound of the formula

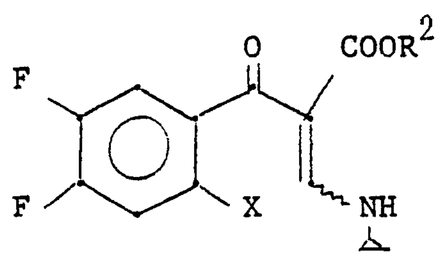

IX

wherein $R^2$ is methyl and X is fluoro, chloro or bromo with a base.

2. A process according to claim 1, characterized in that said compound of the formula IX is prepared by reacting a compound of the formula

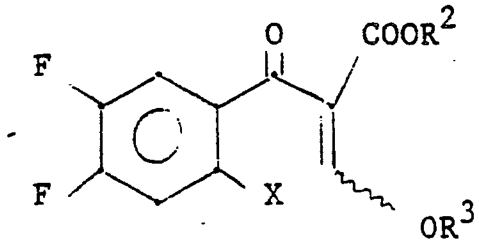

VIII

wherein $R^2$ is methyl, $R^3$ is methyl or ethyl and X is fluoro, chloro, bromo, with cyclopropylamine.

3. A process according to claim 2, characterized in that said compound of the formula VIII is prepared by reacting a compound of the formula:

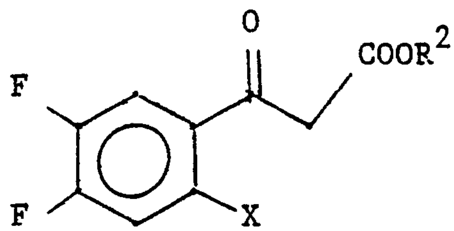

VII

wherein $R^2$ is methyl and X is fluoro, chloro or bromo, with tri($C_1$-$C_4$)alkylorthoformate in the presence of a $C_1$-$C_4$ alkanoic anhydride.

4. A process for preparing a compound of the formula:

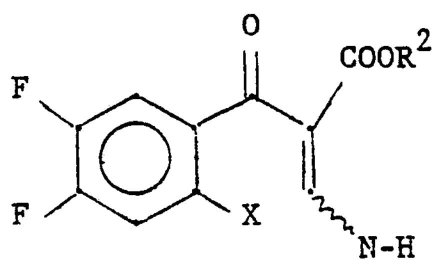

IX

wherein $R^2$ is $C_1$-$C_4$ alkyl, and X is fluoro, chloro or bromo with the proviso that X can be fluoro only when $R^2$ is methyl, characterized by reacting a compound of the formula

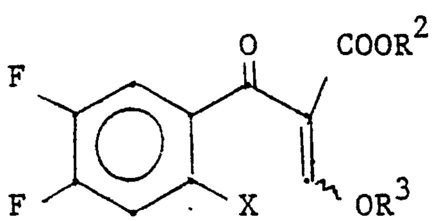

VIII

wherein $R^2$ and X are as defined above and $R^3$ is $C_1$-$C_4$ alkyl, with cyclopropylamine in a $C_5$-$C_7$ cycloalkane to produce said compound of the formula VIII as a crystalline precipitate.

5. A process for preparing a compound of the formula

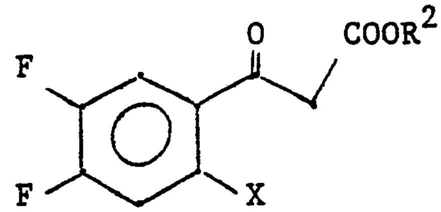

VII

wherein $R^2$ is $C_1$-$C_4$ alkyl and X is fluoro, chloro or bromo, with the proviso that X can be fluoro only when $R^2$ is methyl, characterized by reacting a compound of the formula

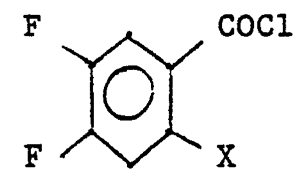

VI

wherein X is as defined above with the dilithium salt of a mono($C_1$-$C_4$)alkyl malonate.

6. A process for preparing a compound of the formula:

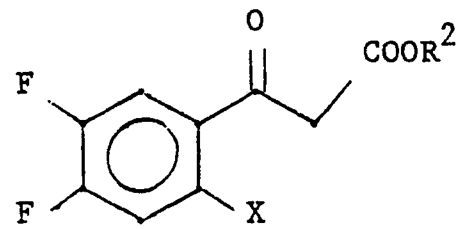

VII

wherein $R^2$ is $C_1$-$C_4$ alkyl and X is fluoro, chloro or bromo, with the proviso that X can be fluoro only when $R^2$ is methyl, characterized by reacting a compound of the formula:

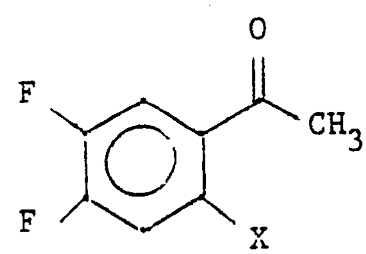

V

wherein X is as defined above with a strong base and then reacting the resulting anion with $C_1$-$C_4$ alkyl cyanoformate.

7. A process for preparing a compound of the formula:

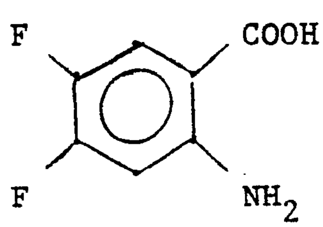

characterized by reacting 5,6-difluoro isatin with alkaline hydrogen peroxide.

8. A process for preparing monomethyl malonic acid or monoethyl malonic acid characterized by reacting a salt of monomethyl malonic acid or monoethyl malonic acid with a mineral acid in water and continuously extracting the product from the aqueous layer with an organic solvent.

9. A process for generating the dianion of a mono($C_1$-$C_4$)alkyl malonic acid characterized by reacting said mono($C_1$-$C_4$)alkyl malonic acid with n-butyl lithium in an inert solvent at a temperature of about -20 to about 20° C.